# EUROPÄISCHE PATENTSCHRIFT

(11) **EP 1 673 322 B1**
(45) Veröffentlichungstag und Bekanntmachung des Hinweises auf die Patenterteilung: **16.04.2008**
(21) Anmeldenummer: 04765659.0
(22) Anmeldetag: 28.09.2004
(51) Int. Cl.: C07C 13/66

(54) **SYNTHESE VON PHENYLSUBSTITUIERTEN FLUORANTHENEN DURCH DIELS-ALDER-REAKTION UND IHRE VERWENDUNG**
SYNTHESIS OF PHENYL-SUBSTITUTED FLUORANTHENES BY A DIESEL-ALDER REACTION AND THE USE THEREOF
SYNTHESE DE FLUORANTHENES SUBSTITUES PAR UN PHENYLE PAR UNE REACTION DIELS-ALDER ET LEUR UTILISATION

(30) Priorität: 29.09.2003 DE 10345583
(43) Veröffentlichungstag der Anmeldung: 28.06.2006
(73) Patentinhaber: BASF SE, 67056 Ludwigshafen (DE)
(72) Erfinder: DÖTZ, Florian, 69120 Heidelberg (DE); SCHWALM, Reinhold, 67157 Wachenheim (DE); RÖSCH, Joachim, 67063 Ludwigshafen (DE)
(74) Vertreter: Isenbruck, Günter
(86) Internationale Anmeldenummer: PCT/EP2004/010850
(87) Internationale Veröffentlichungsnummer: WO 2005/033051

(56) Entgegenhaltungen:
- PATENT ABSTRACTS OF JAPAN Bd. 2000, Nr. 26, 1. Juli 2002 (2002-07-01) & JP 2001 257075 A (MITSUI CHEMICALS INC), 21. September 2001 (2001-09-21)
- GHOSH K ET AL: "SYNTHESIS OF ARYLATED FLUORANTHENE DERIVATIVES" INDIAN JOURNAL OF CHEMISTRY, JODHPUR, IN, Bd. 16B, Nr. 2, 1. Februar 1978 (1978-02-01), Seiten 152-153, XP000570586

## Beschreibung

Die vorliegende Erfindung betrifft Fluoranthenderivate, Verfahren zu ihrer Herstellung sowie die Verwendung von Fluoranthenderivaten als Emittermolekül in organischen lichtemittierenden Dioden (OLEDs), eine lichtemittierende Schicht enthaltend die erfindungsgemäßen Fluoranthenderivate als Emittermoleküle, eine OLED enthaltend die erfindungsgemäße lichtemittierende Schicht sowie Vorrichtungen enthaltend die erfindungsgemäße OLED.

In organischen lichtemittierenden Dioden (OLED) wird die Eigenschaft von Materialien ausgenutzt, Licht zu emittieren, wenn sie durch elektrischen Strom angeregt werden. OLEDs sind insbesondere interessant als Alternative zu Kathodenstrahlröhren und Flüssigkristalldisplays zur Herstellung von Flachbildschirmen.

Es wurden zahlreiche Materialien vorgeschlagen, die bei Anregung durch elektrischen Strom Licht emittieren.

Eine Übersicht über organische lichtemittierende Dioden ist z. B. in M. T. Bernius et al., Adv. Mat. 2000, 12, 1737 offenbart. Die Anforderungen an die verwendeten Verbindungen sind dabei hoch und es gelingt mit den bekannten Materialien üblicherweise nicht, alle gestellten Anforderungen zu erfüllen.

In US 5,281,489 sind OLEDs offenbart, die als fluoreszierende Materialien unter anderem 3,4-Benzofluoranthen oder monomeres unsubstituiertes Fluoranthen enthalten können. Monomeres unsubstituiertes Fluoranthen kann jedoch unter den in den OLEDs herrschenden Anwendungsbedingungen migrieren. Die Schicht aus monomerem unsubstituierten Fluoranthen ist nicht stabil, woraus eine geringe Lebensdauer der Dioden resultiert.

Der Einsatz spezieller Fluoranthenderivate ist in US 2002/0022151 A1 und EP-A 1 138 745 offenbart.

EP-A 1 138 745 betrifft eine OLED, die rötliches Licht emittiert. Diese OLED enthält eine organische Schicht, die eine Verbindung mit einem Fluoranthengerüst aufweist, wobei das Fluoranthengerüst mit mindestens einer Aminogruppe oder einer Alkenylgruppe substituiert ist. Bevorzugt sind gemäß der Beschreibung solche Fluoranthenderivate, die mindestens 5, bevorzugt mindestens 6 kondensierte Ringe aufweisen. Diese Verbindungen emittieren Licht längerer Wellenlänge, so dass gelbes bis rötliches Licht emittiert werden kann. Die in EP-A 1 138 745 offenbarten Fluoranthenderivate tragen bevorzugt eine Aminogruppe zur Erhöhung der Lebensdauer der Fluoranthenderivate.

US 2002/0022151 A1 betrifft ebenfalls OLEDs, die als lichtemittierendes Material spezielle Fluoranthenverbindungen enthalten. Diese Fluoranthenverbindungen weisen mindestens eine Diarylaminogruppe auf.

JP-A 10-169992 betrifft Benzofluoranthenderivate und deren Einsatz unter anderem in organischen lichtemittierenden Dioden. Die Benzofluoranthenderivate gemäß JP-A 10-169992 zeigen ein Absorptionsmaximum bei etwa 410 nm, das heißt im blau-violetten Bereich.

In JP 2001-244075 A sind Fluoranthenderivate und organische Leuchtdioden, die diese aufweisen, offenbart. Die Fluoranthenderivate weisen in ihrer 3-Position einen mit einem weiteren Fluoranthenrest substituierten Naphthalinrest auf.

Aufgabe der vorliegenden Erfindung ist es, Verbindungen bereit zu stellen, die als Emittermoleküle in OLEDs geeignet sind, die ein lange Lebensdauer aufweisen, in OLEDs hocheffizient sind, ein Emissionsmaximum im blauen Bereich und eine hohe Quantenausbeute aufweisen.

Diese Aufgabe wird gelöst durch Fluoranthenderivate der allgemeinen Formel I 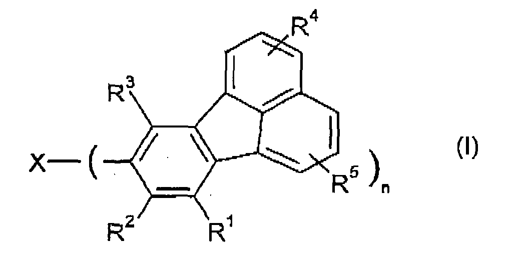 worin die Symbole die folgenden Bedeutungen aufweisen
- R¹, R², R³, R⁴, R⁵: Wasserstoff, Alkyl, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
- X: Alkyl, ein aromatischer Rest, ein kondensiertes aromatisches Ringsystem, ein heteroaromatischer Rest oder ein Rest der Formel (I') 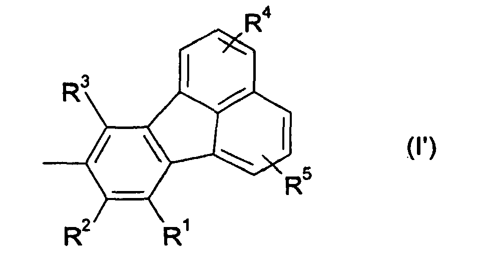 oder eine Oligophenylgruppe;
- n: 2 oder 3, im Falle von X=Oligophenylgruppe, 1 - 20;
unter der Bedingung, dass R¹, R², R³ und X nicht gleichzeitig Phenyl bedeuten, wenn R⁴ und R⁵ Wasserstoff sind.

Die erfindungsgemäßen Fluoranthenderivate weisen Substituenten auf, die über eine C-C-Einfachbindung mit dem Fluoranthengerüst verknüpft sind. Die erfindungsgemäßen Fluoranthenderivate sind überraschenderweise hinreichend stabil, um in einer lichtemittierenden Schicht in OLEDs mit einer langen Lebensdauer eingesetzt zu werden. Des Weiteren zeichnen sich die erfindungsgemäßen Verbindungen durch eine sehr hohe Stabilität gegenüber Photooxidation bei der Verwendung in OLEDs aus.

Es wurde überraschenderweise gefunden, dass die erfindungsgemäßen Fluoranthenderivate Licht in der blauen Region des sichtbaren elektromagnetischen Spektrums emittieren. Das heißt, die erfindungsgemäßen Fluoranthenderivate emittieren im Allgemeinen Licht in der Region des sichtbaren elektromagnetischen Spektrums von 430 bis 480 nm, bevorzugt 440 bis 470 nm, besonders bevorzug 450 bis 470 nm.

Zur Herstellung von Displays, die die Farben des gesamten sichtbaren Spektrums umfassen, ist es erforderlich, OLEDs bereit zu stellen, die Licht in der roten Region des sichtbaren elektromagnetischen Spektrums emittieren, OLEDs, die in der grünen Region des sichtbaren elektromagnetischen Spektrums Licht emittieren und OLEDs, die in der blauen Region des sichtbaren elektromagnetischen Spektrums Licht emittieren. Es hat sich herausgestellt, dass insbesondere die Bereitstellung von effizienten OLEDs, die Licht in der blauen Region des sichtbaren elektromagnetischen Spektrums emittieren, problematisch ist.

Die erfindungsgemäßen Fluoranthenderivate sind zur Herstellung von OLEDs, die Licht in der blauen Region des sichtbaren elektromagnetischen Spektrums emittieren, geeignet.

Im Sinne der vorliegenden Anmeldung bedeutet "Alkyl" eine lineare, verzweigte oder zyklische substituierte oder unsubstituierte C₁- bis C₂₀-, bevorzugt C₁- bis C₉-Alkylgruppe. Falls X und R² eine Alkylgruppe darstellen, handelt es sich bevorzugt um eine lineare oder verzweigte C₃- bis C₁₀-, besonders bevorzugt C₅- bis C₉- Alkylgruppe. Die Alkylgruppen können unsubstituiert oder mit aromatischen Resten, Halogen-, Nitro-, Ether- oder Carboxylgruppen substituiert sein. Besonders bevorzugt sind die Alkylgruppen unsubstituiert oder mit aromatischen Resten substituiert. Bevorzugte aromatische Reste sind nachstehend genannt. Des Weiteren können ein oder mehrere nicht benachbarte Kohlenstoffatome der Alkylgruppe, das/die nicht unmittelbar an das Fluoranthengerüst gebunden ist/sind, durch Si, P, O oder S, bevorzugt durch O oder S, ersetzt sein.

Im Sinne der vorliegenden Anmeldung bedeutet "aromatischer Rest" bevorzugt eine C₆-Arylgruppe (Phenylgruppe). Diese Arylgruppe kann unsubstituiert oder mit linearen, verzweigten oder zyklischen C₁- bis C₂₀-, bevorzugt C₁- bis C₉-Alkylgruppen, die wiederum mit Halogen-, Nitro-, Ether- oder Carboxylgruppen substituiert sein können oder mit einer oder mehreren Gruppen der Formel I', substituiert sein. Des Weiteren können ein oder mehrere Kohlenstoffatome der Alkylgruppe durch Si, P, O, S oder N, bevorzugt O oder S, ersetzt sein. Des Weiteren können die Arylgruppen oder die Heteroarylgruppen mit Halogen-, Nitro-, Carboxylgruppen, Aminogruppen oder Alkoxygruppen oder C₆- bis C₁₄-, bevorzugt C₆- bis C₁₀-Arylgruppen, insbesondere Phenyl- oder Naphthylgruppen, substituiert sein. Besonders bevorzugt bedeutet "aromatischer Rest" eine C₆-Arylgruppe, die gegebenenfalls einer oder mehreren Gruppen der Formel I', mit Halogen, bevorzugt Br, Cl oder F, Aminogruppen, bevorzugt NAr'Ar", wobei Ar' und Ar" unabhängig voneinander C₆-Arylgruppen bedeuten, die, wie vorstehend definiert, unsubstituiert oder substituiert sein können und die Arylgruppen Ar' und Ar" neben den vorstehend genannten Gruppen auch jeweils mit mindestens einem Rest der Formel I' substituiert sein können; und/oder Nitrogruppen substituiert ist. Ganz besonders bevorzugt ist diese Arylgruppe unsubstituiert oder mit NAr'Ar" substituiert.

Im Sinne der vorliegenden Anmeldung bedeutet "kondensiertes aromatisches Ringsystem" ein kondensiertes aromatisches Ringsystem mit im Allgemeinen 10 bis 20 Kohlenstoffatomen, bevorzugt 10 bis 14 Kohlenstoffatomen. Diese kondensierten aromatischen Ringsysteme können unsubstituiert oder mit linearen, verzweigten oder zyklischen C₁- bis C₂₀-, bevorzugt C₁- bis C₉-Alkylgruppen, die wiederum mit Halogen-, Nitro-, Ether- oder Carboxylgruppen substituiert sein können, substituiert sein. Des Weiteren können ein oder mehrere Kohlenstoffatome der Alkylgruppe durch Si, P, O, S oder N, bevorzugt O oder S, ersetzt sein. Des Weiteren können die kondensierten aromatischen Gruppen mit Halogen-, Nitro-, Carboxylgruppen, Aminogruppen oder Alkoxygruppen oder C₆- bis C₁₄-, bevorzugt C₆- bis C₁₀-Arylgruppen, insbesondere Phenyl- oder Naphthylgruppen, substituiert sein. Besonders bevorzugt bedeutet "kondensiertes aromatisches Ringsystem" ein kondensiertes aromatisches Ringsystem, das gegebenenfalls mit Halogen, bevorzugt Br, Cl oder F, Aminogruppen, bevorzugt NAr'Ar", wobei Ar' und Ar" unabhängig voneinander C₆-Arlygruppen bedeuten, die, wie vorstehend definiert, unsubstituiert oder substituiert sein können und die Arylgruppen Ar' und Ar" neben den vorstehend genannten Gruppen auch jeweils mit mindestens einem Rest der Formel I' substituiert sein können, oder Nitrogruppen substituiert ist. Ganz besonders bevorzugt ist das kondensierte aromatische Ringsystem unsubstituiert. Geeignete kondensierte aromatische Ringsysteme sind zum Beispiel Naphthalin, Anthracen, Pyren, Phenanthren oder Perylen.

Im Sinne der vorliegenden Anmeldung bedeutet "heteroaromatischer Rest" eine C₄- bis C₁₄-, bevorzugt C₄- bis C₁₀-, besonders bevorzugt C₄- bis C₅-Heteroarylgruppe, enthaltend mindestens ein N- oder S-Atom. Diese Heteroarylgruppe kann unsubstituiert oder mit linearen, verzweigten oder zyklischen C₁- bis C₂₀-, bevorzugt C₁- bis C₉-Alkylgruppen, die wiederum mit Halogen-, Nitro-, Ether- oder Carboxylgruppen substituiert sein können, substituiert sein. Des Weiteren können ein oder mehrere Kohlenstoffatome der Alkylgruppe durch Si, P, O, S oder N, bevorzugt O oder S, ersetzt sein. Des Weiteren können die Heteroarylgruppen mit Halogen-, Nitro-, Carboxylgruppen, Aminogruppen oder Alkoxygruppen oder C₆- bis C₁₄-, bevorzugt C₆- bis C₁₀-Arylgruppen, substituiert sein. Besonders bevorzugt bedeutet "heteraromatischer Rest" eine Heteroarylgruppe, die gegebenenfalls mit Halogen, bevorzugt Br, Cl oder F, Aminogruppen, bevorzugt NArAr', wobei Ar und Ar' unabhängig voneinander C₆-Arlygruppen bedeuten, die, wie vorstehend definiert, unsubstituiert oder substituiert sein können, oder Nitrogruppen substituiert ist. Ganz besonders bevorzugt ist die Heteroarylgruppe unsubstituiert.

Im Sinne der vorliegenden Anmeldung bedeutet "Oligophenylgruppe" eine Gruppe der allgemeinen Formel (IV) 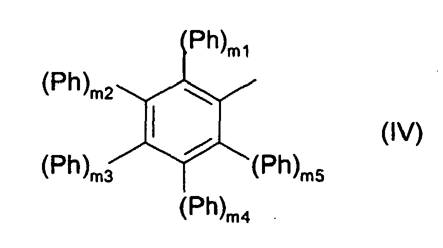 wobei Ph jeweils Phenyl bedeutet, das in allen 5 substituierbaren Positionen jeweils wiederum mit einer Gruppe der Formel (IV) substituiert sein kann;
- m¹ , m² m³ m⁴ und m⁵: bedeuten unabhängig voneinander, 0 oder 1, wobei mindestens ein Index m¹, m², m³, m⁴ oder m⁵ mindestens 1 bedeutet.

Bevorzugt sind Oligophenyle, worin m¹, m³ und m⁵ 0 bedeuten und m² und m⁴ 1 oder Oligophenyle, worin m¹, m², m⁴ und m⁵ 0 bedeuten und m³ 1 bedeutet, sowie Oligophenyle, worin m² und m⁴ 0 bedeuten und m¹, m⁵ und m³ 1 bedeuten.

Die Oligophenylgruppe kann somit eine dendritische, das heißt hyperverzweigte Gruppe sein, insbesondere wenn m¹, m³ und m⁵ 0 bedeuten und m² und m⁴ 1 bedeuten oder wenn m² und m⁴ 0 bedeuten und m¹, m³ und m⁵ 1 bedeuten und die Phenylgruppen wiederum in 1 bis 5 ihrer substituierbaren Positionen mit einer Gruppe der Formel (IV) substituiert sind, bevorzugt in 2 oder 3 Positionen, besonders bevorzugt - im Falle einer Substitution in 2 Positionen - jeweils in meta-Position zur Verknüpfungsstelle mit dem Grundkörper der Formel (IV) und - im Falle einer Substitution in 3 Positionen - jeweils in ortho-Position und in para-Position zur Verknüpfungsstelle mit dem Grundkörper der Formel (IV).

Die Oligophenylgruppe kann jedoch auch im Wesentlichen unverzweigt sein, insbesondere dann, wenn nur einer der Indices m¹, m², m³, m⁴ oder m⁵ 1 ist, wobei im unverzweigten Fall bevorzugt m³ 1 ist und m¹, m², m⁴ und m⁵ 0 sind. Die Phenylgruppe kann wiederum in 1 bis 5 ihrer substituierbaren Positionen mit einer Gruppe der Formel (IV) substituiert sein, bevorzugt ist die Phenylgruppe an 1 ihrer substituierbaren Positionen mit einer Gruppe der Formel (IV) substituiert, besonders bevorzugt in para-Position zur Verknüpfungsstelle mit dem Grundkörper. Im Folgenden werden die direkt mit dem Grundkörper verknüpften Substituenten erste Substituenten-Generationen genannt. Die Gruppe der Formel (IV) kann wiederum wie vorstehend definiert substituiert sein. Im Folgenden werden die mit der ersten Substituenten-Generation verknüpften Substituenten zweite Substituenten-Generation genannt.

Entsprechend der ersten und zweiten Substituenten-Generation sind beliebig viele weitere Substituenten-Generationen möglich. Bevorzugt sind Oligophenylgruppen mit den vorstehend genannten Substitutionsmustern, die eine erste und eine zweite Substituenten-Generation aufweisen oder Oligophenylgruppen, die lediglich eine erste Substituenten-Generation aufweisen.

Unter einer "Oligophenylgruppe" im Sinne der vorliegenden Anmeldung sind des Weiteren solche Gruppen zu verstehen, die auf einem Grundkörper gemäß einer der Formeln V, VI oder VII basieren: 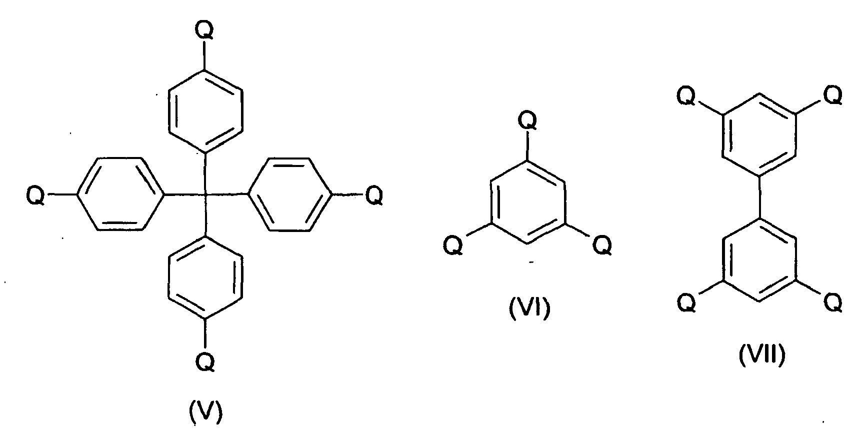 wobei Q jeweils eine Bindung zu einem Rest der Formel I' oder eine Gruppe der Formel VIII bedeutet: 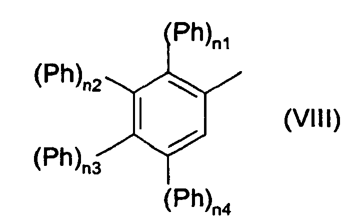 wobei Ph jeweils Phenyl bedeutet, das in maximal 4 Positionen entsprechend dem Substitutionsmuster des zentralen Phenylrings der Gruppe der Formel VIII wiederum mit einer Gruppe der Formel VIII substituiert sein kann;
- n¹, n², n³ und n⁴: bedeuten unabhängig voneinander 0 oder 1, wobei bevorzugt n¹, n², n³ und n⁴ 1 bedeuten.

Die Oliophenylgruppen der Formeln V, VI und VII können somit dendritische, das heißt hyperverzweigte Gruppen sein.

Die Oligophenyle der Formeln IV, V, VI und VII sind mit 1 bis 20, bevorzugt 4 bis 16, besonders bevorzugt 4 bis 8 Resten der Formel (I') substituiert, wobei ein Phenylrest mit einem, keinem oder mehreren Resten der Formel (I') substituiert sein kann. Bevorzugt ist ein Phenylrest mit einem oder keinem Rest der Formel (I') substituiert, wobei mindestens ein Phenylrest mit einem Rest der Formel (I') substituiert ist.

Ganz besonders bevorzugte Verbindungen der Formel I, worin X ein Oligophenylrest der allgemeinen Formel IV ist, sind im Folgenden genannt:

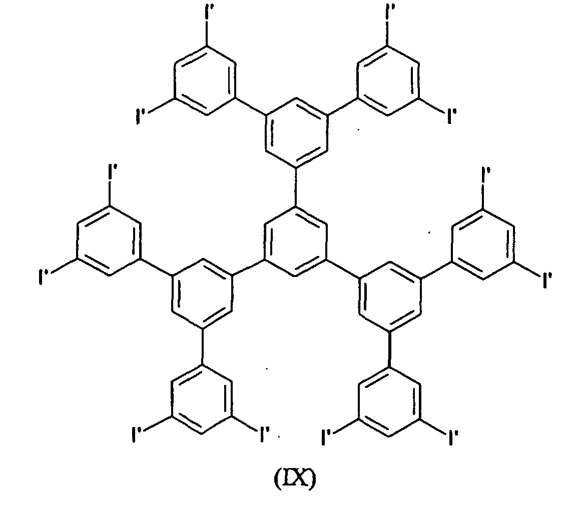

Ganz besonders bevorzugte Verbindungen der Formel I, worin X ein Oligophenylrest der allgemeinen Formeln V, VI oder VII ist, sind im Folgenden genannt:

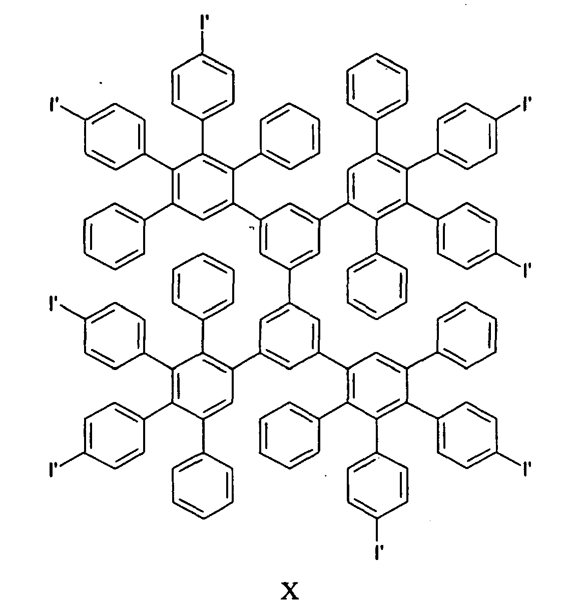

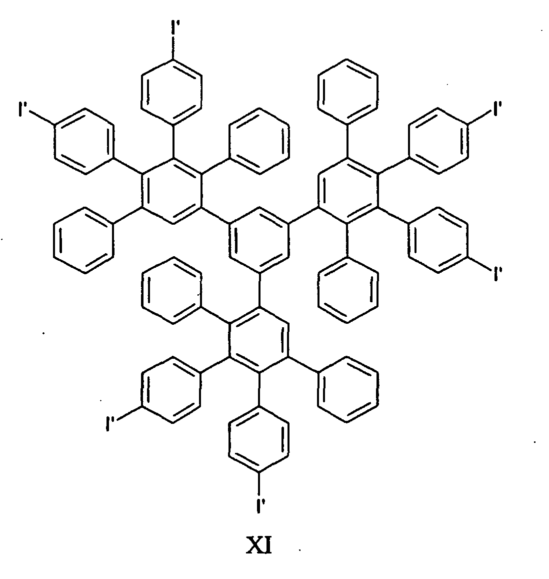

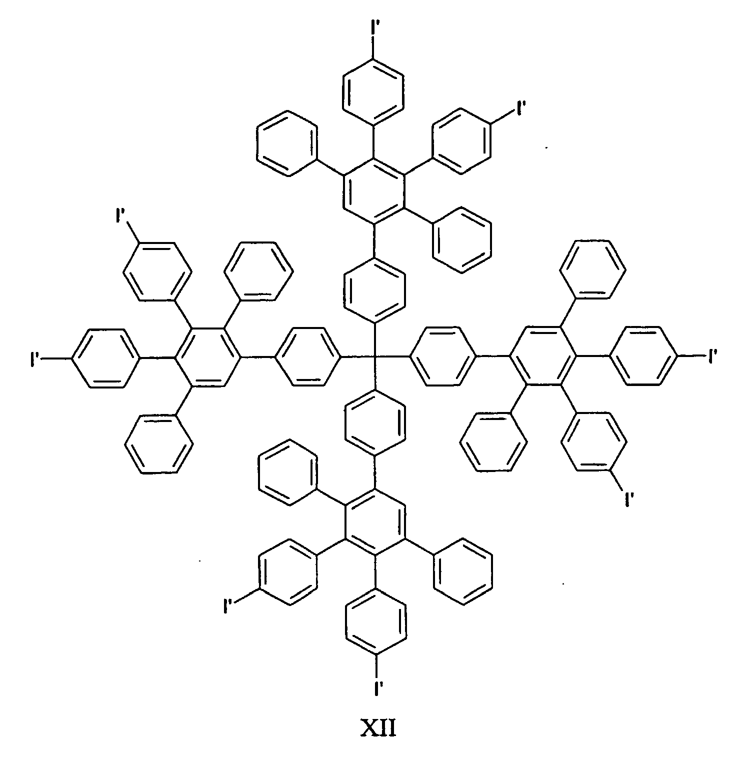

Die Reste R¹, R², R³, R⁴, R⁵ und X können unabhängig voneinander aus den vorstehend genannten Resten ausgewählt sein, mit der Maßgabe, dass mindestens einer der Reste R¹, R² und/oder R³ nicht Wasserstoff ist und R¹, R², R³ und X nicht gleichzeitig Phenyl bedeuten, wenn R⁴ und R⁵ Wasserstoff sind.

R⁴ und R⁵ sind bevorzugt Wasserstoff.

R¹ und R³ sind bevorzugt ein aromatischer Rest, ein kondensiertes aromatisches Rinsystem oder ein Rest der Formel I', besonders bevorzugt ein aromatischer Rest, wobei bevorzugte Ausführungsformen des aromatischen Rests bereits vorstehend aufgeführt sind. Ganz besonders bevorzugt sind R¹ und R³ Phenyl.

R² ist bevorzugt Wasserstoff, Alkyl, wobei bevorzugte Ausführungsformen des Alkylrests bereits vorstehend genannt sind, besonders bevorzugt C₁- bis C₉- Alkyl, das ganz besonders bevorzugt unsubstituiert und linear ist, ein aromatischer Rest, wobei bevorzugte aromatische Reste bereits vorstehend genannt sind, besonders bevorzugt ein Phenylrest.

X ist bevorzugt ein aromatischer Rest, wobei bevorzugte aromatische Reste bereits vorstehend genannt sind, besonders bevorzugt ein C₆-Arlyrest, der in Abhängigkeit von n ein- bis dreifach mit Fluoranthenylresten substituiert ist, oder ein kondensiertes aromatisches Ringsystem, wobei bevorzugte kondensierte aromatische Ringsysteme bereits vorstehend genannt sind, besonders bevorzugt ein C₁₀- bis C₁₄- kondensiertes aromatisches Ringsystem, ganz besonders bevorzugt Naphthyl oder Anthracenyl, wobei das kondensierte aromatische Ringsystem in Abhängigkeit von n ein- bis dreifach mit Fluoranthenylresten substituiert ist. Ist X ein aromatischer Rest mit 6 Kohlenstoffatomen, so ist dieser Rest bevorzugt in 1- und 4-Position oder in 1-, 3- und 5-Position mit Fluoranthenylresten substituiert. Ist X zum Beispiel ein Anthracenylrest, so ist dieser bevorzugt in 9- und 10-Position mit Fluoranthenylresten substituiert. Unter Fluoranthenylresten sind dabei Gruppierungen der nachfolgend aufgeführten Formel I' zu verstehen

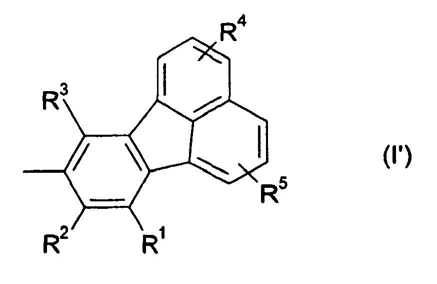

Es ist auch möglich, dass der Rest X selbst ein Fluoranthenylrest der Formel I' ist.

Des weiteren kann X eine Oligophenylgruppe sein, wobei bevorzugte Oligophenylgruppen bereits vorstehend genannt sind. Bevorzugt ist eine Oligophenylgruppe der allgemeinen Formel (IV), worin m¹, m², m³, m⁴ und m⁵ 0 oder 1 sind, wobei mindestens einer der Indices m¹, m², m³, m⁴ oder m⁵ 1 ist.

n ist eine ganz Zahl von 2 oder 3. Das bedeutet, die Fluoranthenderivate der allgemeinen Formel I weisen bevorzugt mehr als einen Fluoranthenylrest der allgemeinen Formel I' auf. Somit sind ebenfalls solche Verbindungen bevorzugt, in denen X selbst ein Fluoranthenylrest ist. Wenn X eine Oligophenylgruppe ist, ist n eine ganze Zahl von 1 bis 20, bevorzugt 4 bis 16.

Ganz besonders bevorzugt sind Fluoranthenderivate der allgemeinen Formel I, die keine Heteroatome aufweisen.

In einer ganz besonders bevorzugten Ausführungsform ist X ein gegebenenfalls substituierter Phenylrest und n 2 oder 3. Das bedeutet, der Phenylrest ist mit 2 oder 3 Resten der Formel I' substituiert. Bevorzugt enthält der Phenylrest keine weiteren Substituenten. Wenn n 2 ist, befinden sich die Reste der Formel I' jeweils in para-Position zueinander. Ist n 3, befinden sich die Reste jeweils in meta-Position zueinander.

In einer weiteren bevorzugten Ausführungsform ist X ein gegebenenfalls substituierter Phenylrest und n 1, das heißt, der Phenylrest ist mit einem Rest der Formel I' substituiert.

Weiterhin bevorzugt ist X ein Anthracenylrest und n 2. Das bedeutet, der Anthracenylrest ist mit 2 Resten der Formel I' substituiert. Diese Reste stehen bevorzugt in 9- und 10-Position des Anthracenylrests.

Die Herstellung der erfindungsgemäßen Fluoranthenderivate der allgemeinen Formel I kann nach allen geeigneten dem Fachmann bekannten Verfahren durchgeführt werden. In einer bevorzugten Ausführungsform werden die Fluoranthenderivate der Formel I durch Umsetzung von Cyclopentaacenaphthenonderivaten (im Folgenden Acecyclonderivate genannt) hergestellt. Geeignete Herstellungsverfahren für Verbindungen der Formel I, worin n 1 ist, sind zum Beipiel in Dilthey et al., Chem. Ber. 1938, 71, 974 und Van Allen et al., J. Am. Chem. Soc., 1940, 62, 656 offenbart.

In einer bevorzugten Ausführungsform erfolgt die Herstellung der erfindungsgemäßen Fluoranthenderivate der allgemeinen Formel I durch Umsetzung von Acecyclonderivaten mit Alkinylverbindungen.

Ein weiterer Gegenstand der vorliegenden Anmeldung ist daher ein Verfahren zur Herstellung der erfindungsgemäßen Fluoranthenderivate durch Umsetzung einer Verbindung der Formel (II) 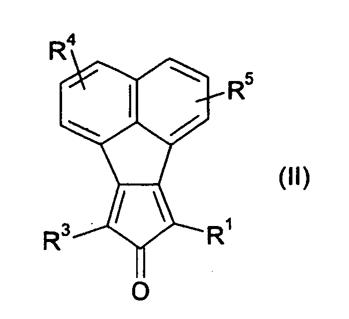 mit einer Alkinylverbindung der Formel (III) 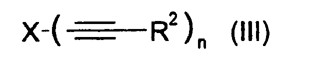 und anschließender Kohlenmonoxideliminierung,
worin die Symbole die folgenden Bedeutungen aufweisen:
- R¹, R ², R ³, R ⁴, R⁵: Wasserstoff, Alkyl, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
- X: Alkyl, ein aromatischer Rest, ein kondensiertes aromatisches Ringsystem, ein heteroaromatischer Rest oder ein Rest der Formel (I') 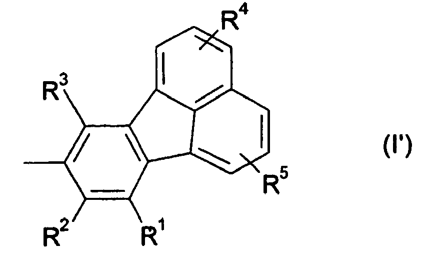 oder eine Oligophenylgruppe; und
- n: 2 oder 3, im Falle von X=Oligophenylgruppe 1 bis 20.

Das Acecyclonderivat der Formel II wird gemäß aus dem Stand der Technik bekannten Verfahren hergestellt, zum Beispiel gemäß einem Verfahren in Dilthey et al., J. prakt. Chem. 1935, 143, 189, worin die Synthese von Acecyclon(7,9-diphenylcyclopenta[a]acenaphthylen-8-on) offenbart ist. Derivate des Acecyclons können in analoger Weise erhalten werden.

Die Alkinylverbindungen der Formel III können ebenfalls nach dem Fachmann bekannten Verfahren hergestellt werden. Geeignete Verfahren sind zum Beispiel in Hagihara et al., Synthesis (1980), 627 und L. Cassar, J. Organomet. Chem. 93 (1979), 253 offenbart.

Das Verhältnis des Acecyclonderivats der Formel II und der Alkinylverbindung der Formel III ist abhängig davon, wie viele Fluoranthenylreste das gewünschte Fluoranthenderivat der Formel I tragen soll, das heißt, das Verhältnis von Acecyclonderivat der Formel II zu der Alkinylverbindung der Formel III ist abhängig von n. Im Allgemeinen werden das Acecyclonderivat (II) und die Alkinylverbindung (III) in einem molaren Verhältnis von n zu 1 bis n + 15 % zu 1, bevorzugt n zu 1 bis n + 10 % zu 1 eingesetzt. Bei n = 1 ist ein etwa äquimolares Verhältnis bevorzugt und bei n > 1 wird bevorzugt mit einem Verhältnis von Acecyclonderivat (II) zu Alkinylverbindung (III) von n + 10 % zu 1 gearbeitet. Geeignete Werte für n wurden vorstehend genannt.

Bei einer Umsetzung von Acecyclonderivaten der Formel II mit einer Alkinylverbindung der Formel III, worin n 1 ist, erfolgt eine Umsetzung in einem molaren Verhältnis des Acecyclonderivats der Formel II zu der Alkinylverbindung der Formel III von im Allgemeinen 1 zu 1 bis 1,3 zu 1, bevorzugt 1 zu 1 bis 1,1 zu 1.

Bei einer Umsetzung des Acecyclonderivats der Formel II mit einer Alkinylverbindung der Formel III, worin n 2 ist, erfolgt die Umsetzung in einem Molverhältnis der Acecyclonverbindung der Formel II zu der Alkinylverbindung der Formel III von im Allgemeinen 2 zu 1 bis 2,5 zu 1, bevorzugt 2,1 zu 1 bis 2,3 zu 1.

Wird die Acecyclonverbindung der Formel II mit einer Alkinylverbindung der Formel III umgesetzt, worin n 3 ist, erfolgt die Umsetzung in einem molaren Verhältnis der Acecyclonverbindung der Formel II zu der Alkinylverbindung der Formel III von im Allgemeinen 3 zu 1 bis 3,5 zu 1, bevorzugt 3,2 zu 1 bis 3,4 zu 1.

Bevorzugte Reste R¹, R³, R⁴ und R⁵ des Acecyclonderivats der Formel II sowie bevorzugte Reste X und R² der Alkinylverbindung der Formel III sowie bevorzugte Indizes n der Alkinylverbindung der Formel III entsprechen den bevorzugten Resten R¹, R², R³, R⁴, R⁵ und X sowie den bevorzugten Indizes n, die bezüglich der erfindungsgemäßen Fluoranthenderivate der allgemeinen Formel I erwähnt wurden.

In einer ganz besonders bevorzugten Ausführungsform sind R⁴ und R⁵ Wasserstoff und R¹ und R³ Phenyl. Ganz besonders bevorzugt wird somit als Acecyclonderivat der Formel II Acecyclon selbst eingesetzt.

Ganz besonders bevorzugt eingesetzte Alkinylverbindungen sind zum Beispiel solche, in denen n 1 ist wie 9-Nonadecin, 1-Octin, 1-Decin und 1-Octadecin, solche, in denen n 2 ist wie 1,4-Diethinylbenzol und 9,10-Bis-phenylethinylanthracen sowie 2,4-Hexadiin, und solche, worin n 3 ist wie 1,3,5-Triethinylbenzol.

Ist X ein Oligophenyl, werden Oligophenylderivate als Alkinylverbindungen eingesetzt, die genau n freie Acetylengruppen (-C≡C-H) tragen.

Bei der Umsetzung gemäß dem erfindungsgemäßen Verfahren handelt es sich um eine Diels-Alder-Reaktion mit anschließender Kohlenmonoxideliminierung.

Die Umsetzung erfolgt im Allgemeinen in einem Lösungsmittel, bevorzugt in einem organischen unpolaren Lösungsmittel, besonders bevorzugt in einem organischen unpolaren Lösungsmittel mit einem Siedepunkt oberhalb von im Allgemeinen 100 °C, bevorzugt oberhalb von 140 °C, besonders bevorzugt oberhalb von 260 °C durchgeführt.

Geeignete Lösungsmittel sind zum Beispiel Toluol, Xylol, Diphenylether, Methylnaphthalin, Mesitylen, Glycole und ihre Ether, Dekalin und Mischungen der genannten Lösungsmittel.

In einer bevorzugten Ausführungsform des erfindungsgemäßen Verfahrens werden das Acecyclonderivat der Formel II und die Alkinylverbindung der Formel III in dem organischen Lösungsmittel zusammen gegeben und auf Temperaturen von im Allgemeinen 140 bis 260 °C, bevorzugt 140 bis 170 °C, bzw. 240 bis 260 °C erhitzt. Die Temperatur ist dabei abhängig von der Reaktivität der Ausgangsstoffe. Terminale Alkine (R² = H in Formel (II)) reagieren im Allgemeinen bei niedrigeren Temperaturen, bevorzugt bei 140 bis 190 °C, besonders bevorzugt 140 bis 170 °C, ganz besonders bevorzugt 140 bis 160 °C, während interne Alkine (R² ≠ H in Formel (III) im Allgemeinen bei höheren Temperaturen reagieren, bevorzugt bei 190 bis 260 °C, bevorzugt 220 bis 260 °C, besonders bevorzugt 240 bis 260 °C. Die Reaktionsdauer beträgt im Allgemeinen 8 bis 30 Stunden. Die Reaktionsdauer hängt von dem sterischen Anspruch von R² sowie von n in Formel III ab. Bevorzugt beträgt die Reaktionsdauer bei n = 1 8 bis 18, besonders bevorzugt 10 bis 16, ganz besonders bevorzugt 14 bis 16 Stunden. Bei n = 2 beträgt die Reaktionsdauer bevorzugt 18 bis 28, besonders bevorzugt 20 bis 26, ganz besonders bevorzugt 22 bis 26 Stunden. Bei n = 3 beträgt die Reaktionsdauer bevorzugt 24 bis 30, besonders bevorzugt 26 bis 30, ganz besonders bevorzugt 28 bis 30 Stunden.

Das erhaltene Reaktionsgemisch wird in einem polaren Lösungsmittel, zum Beispiel in Methanol, Ethanol oder gegebenenfalls in unpolaren Lösungsmitteln wie Cyclohexan gefällt. Bei besonders löslichen Fluoranthenderivaten kann der Fällungsschritt entfallen. Das erhaltene Produkt wird nach dem Fachmann bekannten Methoden aufgearbeitet. Bevorzugt erfolgt die Aufarbeitung durch Säulenchromatographie, besonders bevorzugt an Kieselgel. Als Laufmittel kann jedes geeignete Laufmittel oder Laufmittelgemisch eingesetzt werden. Ganz besonders bevorzugt wird ein Essigester/Cyclohexangemisch eingesetzt.

Die erhaltenen erfindungsgemäßen Fluoranthenderivate der allgemeinen Formel I zeichnen sich durch ein Absorptionsmaximum im ultravioletten Bereich des elektromagnetischen Spektrums und durch ein Emissionsmaximum im blauen Bereich des elektromagnetischen Spektrums aus. Die Quantenausbeute der erfindungsgemäßen Fluoranthenderivate beträgt im Allgemeinen 20 bis 75 % in Toluol. Es wurde gefunden, dass Fluoranthenderivate der allgemeinen Formel I, worin n 2 oder 3 ist, besonders hohe Quantenausbeuten von über 50 % aufweisen.

Die erfindungsgemäßen Fluoranthenderivate zeichnen sich dadurch aus, dass sie geeignet sind, in organischen lichtemittierenden Dioden (OLEDs) elektromagnetische Strahlung im blauen Bereich des sichtbaren elektromagnetischen Spektrums zu emittieren.

Ein weiterer Gegenstand der vorliegenden Erfindung ist daher die Verwendung von Fluoranthenderivaten der allgemeinen Formel (I) 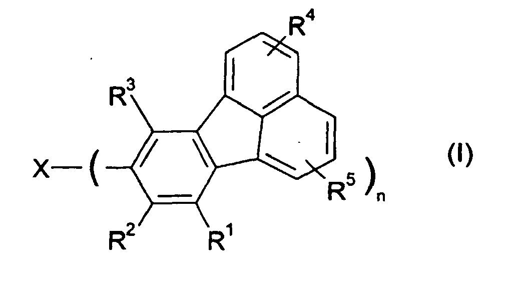 worin die Symbole die folgenden Bedeutungen aufweisen:
- R¹, R², R³, R⁴, R⁵: Wasserstoff, Alkyl, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
- X: Alkyl, ein aromatischer Rest, ein kondensiertes aromatisches Ringsystem, ein heteroaromatischer Rest oder ein Rest der Formel (I') 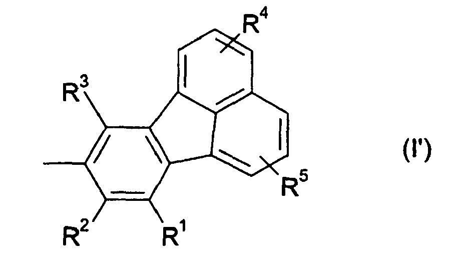 oder eine Oligophenylgruppe;
- n: 2 oder 3, im Falle von X = Oligophenylgruppe, 1 bis 20;
als Emittermolekül in organischen lichtemittierenden Dioden (OLEDs). Bevorzugte Fluoranthenderivate und deren Herstellungsverfahren sind bereits vorstehend genannt.

Organische lichtemittierende Dioden sind grundsätzlich aus mehreren Schichten aufgebaut. Dabei sind verschiedene Schichtenfolgen möglich, z. B.:
- Anode/lochtransportierende Schicht/lichtemittierende Schicht/Kathode;
- Anode/lichtemittierende Schicht/elektronentransportierende Schicht/Kathode;
- Anode/lochtransportierende Schicht/lichtemittierende Schichtlelektronentransportierende Schicht/Kathode.

Die erfindungsgemäßen Fluoranthenderivate der allgemeinen Formel I werden bevorzugt in der lichtemittierenden Schicht als Emittermoleküle eingesetzt. Ein weiterer Gegenstand der vorliegenden Erfindung ist daher eine lichtemittierende Schicht enthaltend ein oder mehrere Fluoranthenderivate der allgemeinen Formel I 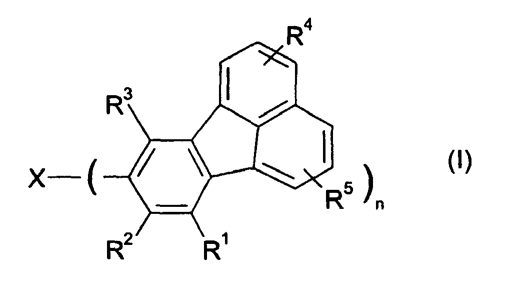 worin die Symbole die folgenden Bedeutungen aufweisen:
- R¹, R², R³, R⁴, R⁵: Wasserstoff, ein Alkylrest, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
- X: ein Alkylrest, ein aromatischer Rest, ein kondensiertes aromatisches Ringsystem, ein heteroaromatischer Rest oder ein Rest der Formel (I') 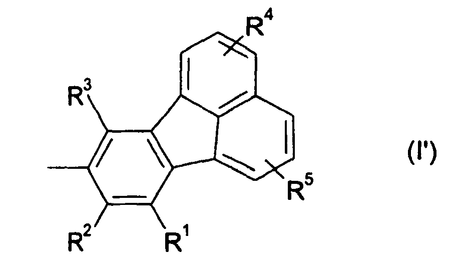 oder eine Oligophenylgruppe;
- n: 2 oder 3, im Fall von X = Oligophenylgruppe, 1 bis 20;
als Emittermoleküle. Bevorzugte Fluoranthenderivate und deren Herstellungsverfahren sind bereits vorstehend genannt.

Die erfindungsgemäßen Fluoranthenderivate können in irgendeiner der vorstehend genannten Schichten ausgewählt aus der lichtemittierenden Schicht, der elektronentransportierenden Schicht und der lochtransportierenden Schicht eingesetzt werden. Bevorzugt werden die erfindungsgemäßen Fluoranthenderivate als Emitter in der lichtemittierenden Schicht eingesetzt. In der lichtemittierenden Schicht werden die Fluoranthenderivate bevorzugt in Substanz, d. h. ohne Zugabe von weiteren Substanzen, eingesetzt. Es ist jedoch auch möglich, zusätzlich zu den erfindungsgemäßen Fluoranthenderivaten übliche lichtemittierende Materialien, Dotierstoffe, lochtransportierende Substanzen und/oder elektronentransportierende Substanzen einzusetzen. Werden die erfindungsgemäßen Fluoranthenderivate nicht in Substanz eingesetzt, so können sie zu irgendeiner der vorstehend genannten Schichten in einer Konzentration von 1 bis 70 Gew.-%, bevorzugt 1 bis 20 Gew.%, zugegeben werden.

Die einzelnen vorstehend genannten Schichten des OLEDs können wiederum aus zwei oder mehreren Schichten aufgebaut sein. Beispielsweise kann die lochtransportierende Schicht aus einer Schicht aufgebaut sein, in die aus der Elektrode Löcher injiziert werden, nachstehend lochinjizierende Schicht genannt, und einer Schicht, die die Löcher von der lochinjizierenden Schicht weg in die lichtemittierende Schicht transportiert. Diese Schicht wird im Folgenden lochtransportierende Schicht genannt. Die elektronentransportierende Schicht kann ebenfalls aus mehreren Schichten bestehen, z. B. aus einer. Schicht, worin Elektronen durch die Elektrode injiziert werden, im Folgenden elektroneninjizierende Schicht genannt, und einer Schicht, die aus der elektroneninjizierenden Schicht Elektronen erhält und in die lichtemittierende Schicht transportiert, im Folgenden elektronentransportierende Schicht genannt. Diese genannten Schichten werden jeweils nach Faktoren wie Energieniveau, Temperaturresistenz und Ladungsträgerbeweglichkeit, sowie Energiedifferenz der genannten Schichten mit den organischen Schichten oder den Metallelektroden ausgewählt.

Geeignete Materialien, die in der lichtemittierenden Schicht als Basismaterial in Kombination mit den erfindungsgemäßen Fluoranthenderivaten der allgemeinen Formel I eingesetzt werden, sind Anthracen, Naphthalin, Phenanthren, Pyren, Tetracen, Coronen, Chrysen, Fluorescein, Perylen, Phthaloperylen, Naphthaloperylen, Perynon, Phthaloperynon, Naphthaloperynon, Diphenylbutadien, Tetraphenylbutadien, Cumarin, Oxadiazol, Aldazin, Bisbenzoxazolin, Bisstyryl, Pyrazin, Cyclopentadien, Metallkomplexe des Chinolins, Metallkomplexe von Aminochinolin, Metallkomplexe des Benzochinolins, Imine, Diphenylethylen, Vinylanthracen, Diaminocarbazol, Pyran, Thiopyran, Polymethin, Merocyanin, Chelate von oxinoiden Verbindungen mit Imidazolen, Chinacridon, Rubren, Stilbenderivate und fluoreszierende Pigmente.

Als lochtransportierendes Material wird im Allgemeinen eine Verbindung eingesetzt, die die Fähigkeit besitzt, die Löcher aus der Anode aufzunehmen, Löcher zu transportieren und gleichzeitig dazu geeignet ist, Löcher in die lichtemittierende Schicht zu injizieren. Geeignete lochtransportierende Materialien sind beispielsweise Metallkomplexe des Phthalocyanins, des Naphthalocyanins, des Porphyrins, Pyrazolone, Tetrahydroimidazole, Hydrazone, Acylhydrazone, Polyarylalkane, Thiophene, tertiäre aromatische Amine wie Triphenylamine des Benzidintyps, Triphenylamine des Styrylamintyps, Triphenylamine des Diamintyps, Derivate dieser Verbindungen Silanamine, insbesondere Silanamine, die Triphenylsilylgruppen tragen, und makromolekulare Verbindungen wie Polyvinylcarbazole, Polyvinylsilane, Polythiophen, Poly (p-Phenylen) und leitfähige Makromoleküle. Besonders bevorzugte lochtransportierende Materialien sind z. B. in EP-A 1 138 745 und Chen et al. Macromol. Symp. 125, 9 bis 15 (1997) offenbart.

Als elektronentransportierende Materialien sind Verbindungen geeignet, die die Fähigkeit aufweisen Elektronen zu transportieren, und die selbst Elektronen in die lichtemittierende Schicht injizieren können. Geeignete elektronentransportierende Materialien sind beispielsweise Oxazole, Oxadiazole, Triazole, Imidazole, Imidazolone, Imidazolethione, Fluorenon, Anthrachinondimethan, Diphenochinon, Thiopyrandioxid, Perylentetracarbonsäure, Fluorenylidenmethan, Distyrylarylene, Arylene, Cumarine und Derivate der genannten Verbindungen sowie Metallchelate. Insbesondere geeignet sind A1Q₃ (Tris(8-hydroxychinolato)aluminium), BeBq₂, 1,3,4-Oxidazolderivate (OXDs) wie PBD und 1,2,4-Triazole (TAZs). Desweiteren sind Bis(benzimidazolyl)-Derivate von Peryldendicarboximid (PD), Naphthalindicarboximid (ND) und Thiopyransulfone (TPS) geeignet. Bevorzugt eingesetzte elektronentransportierende Materialien sind z. B. in EP-A 1 138 745 offenbart.

Um die Stabilität des erfindungsgemäßen OLEDs gegenüber Temperatur, Feuchtigkeit und anderen Einflüssen zu erhöhen, kann das OLED durch eine Schutzschicht auf der Oberfläche des OLED geschützt sein, wobei diese Schutzschicht z. B. aus einem Harz oder Silikonöl aufgebaut ist.

Als leitendes Material, das für die Anode des erfindungsgemäßen OLEDs geeignet ist, wird bevorzugt Material eingesetzt, das eine Arbeitsfunktion von ≥ 4 eV aufweist. Geeignete Materialien für die Anode sind z. B. Kohlenstoff, Vanadium, Eisen, Kobalt, Nickel, Wolfram, Gold, Platin, Palladium und Legierungen dieser Materialien, Metalloxide wie sie für ITO-Substrate (ITO = Indium-Zinn-Oxid) und NESA-Substrate verwendet werden, wie Zinnoxide und Indiumoxide und organische leitende Polymere wie Polythiophen und Polypyrrol.

Als leitendes Material für die Kathode ist Material geeignet, das eine Arbeitsfunktion von < 4 eV aufweist. Für die Kathode geeignete Materialien sind beispielsweise Magnesium, Kalzium, Zinn, Blei, Titan, Yttrium, Lithium, Ruthenium, Mangan, Aluminium und Legierungen dieser Materialien.

Die Anode und die Kathode können gegebenenfalls eine Multischichtstruktur aus zwei oder mehreren Schichten aufweisen.

Die OLEDs der vorliegenden Erfindung weisen bevorzugt zusätzlich eine Schicht eines Chalkogenids, eines Metallhalogenids oder eines Metalloxids auf der Oberfläche mindestens eines Elektrodenpaares auf Besonders bevorzugt ist eine Schicht eines Chalkogenids (inklusive eines Oxids) eines Metalls, z. B. Silizium oder Aluminium, auf die Oberfläche der Anode auf der Seite aufgebracht, die in die Richtung der lichtemittierenden Schicht zeigt. Auf die Oberfläche der Kathode, die in die Richtung der lichtemittierenden Schicht zeigt, wird bevorzugt eine Schicht eines Metallhalogenids oder eines Metalloxids aufgebracht. Aufgrund der beiden oben genannten Schichten kann die Stabilität der OLEDs verbessert werden. Bevorzugte Materialien für die genannten Schichten sind z. B. in EP-A 1 138 745 genannt.

Weitere bevorzugte Ausführungsformen der einzelnen Schichten der OLEDs sind ebenfalls in EP-A 1 138 745 aufgeführt.

Im Allgemeinen ist mindestens eine Seite der erfindungsgemäßen OLED in dem Wellenlängenbereich, in dem Licht emittiert werden soll, transparent, um eine effiziente Lichtemission zu ermöglichen. Die transparente Elektrode wird im Allgemeinen durch vapor deposition oder Sputtering aufgebracht. Bevorzugt weist die Elektrode auf der lichtemittierenden Seite des OLEDs eine Durchlässigkeit für Licht von ≥ 10% auf. Geeignete Materialien sind dem Fachmann bekannt. Beispielsweise können Glas-Substrate oder transparente Polymerfilme eingesetzt werden.

Die Herstellung der erfindungsgemäßen OLEDs ist dem Fachmann bekannt. Es ist möglich, dass jede Schicht der OLED durch ein trockenes Verfahren zur Filmbildung wie vapor deposition, Sputtering, Plasma plating oder Ion plating oder ein nasses Verfahren der Filmbildung wie Spincoating, Tauchen oder Flowcoating hergestellt wird. Die Dicke der einzelnen Schichten ist nicht begrenzt und übliche Dicken sind dem Fachmann bekannt. Geeignete Dicken der Schichten liegen im Allgemeinen im Bereich von 5 nm bis 10 µm. Bevorzugt sind Dicken von 10 nm bis 0,2 µm. Die Durchführung von trockenen Verfahren oder nassen Verfahren zur Filmbildung sind dem Fachmann bekannt.

Ein weiterer Gegenstand der vorliegenden Erfindung ist somit eine OLED enthaltend eine lichtemittierende Schicht, die ein oder mehrere Fluoranthenderivate der allgemeinen Formel (I) 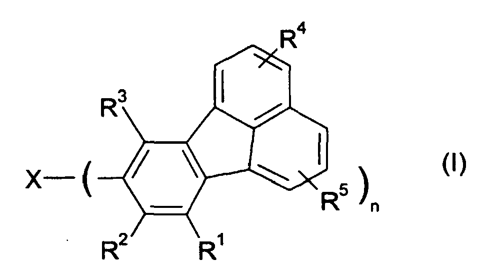 worin die Symbole die folgenden Bedeutungen aufweisen:
- R¹, R², R³, R⁴, R⁵: Wasserstoff, Alkyl, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
- X: Alkyl, ein aromatischer Rest, ein kondensiertes aromatisches Ringsystem, ein heteroaromatischer Rest oder ein Rest der Formel (I') 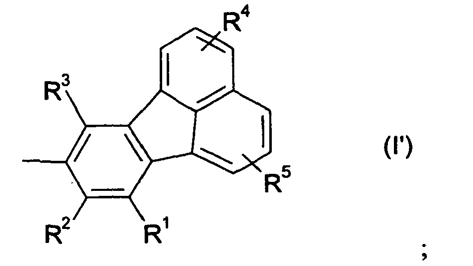 oder eine Oligophenylgruppe
- n: 2 oder 3, im Falle von X = Oligophenylgruppe, 1 bis 20;
als Emittermoleküle enthält. Bevorzugte Verbindungen der allgemeinen Formel 1 sowie deren Herstellungsverfahren sind bereits vorstehend genannt.

Die erfindungsgemäße OLED kann in zahlreichen Vorrichtungen eingesetzt werden. Somit ist ein weiterer Gegenstand der vorliegenden Erfindung eine Vorrichtung ausgewählt aus der Gruppe bestehend aus stationären Bildschirmen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Fahrzeugen sowie Zielanzeigen an Bussen und Bahnen.

Die nachfolgenden Beispiele erläutern die Erfindung zusätzlich.

### Beispiele

### 7,8,9,10-Tetraphenyl-fluoranthen: (nicht erfindungsgemäß)

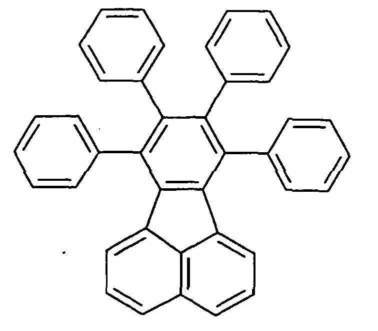

7,8,9,10-Tetraphenyl-fluoranthen wurde synthetisiert gemäß Dilthey et al., Chem. Ber. 1938, 71, 974 und Van Allen et al., J. Am. Chem. Soc., 1940, 62, 656.
λ_{max,em} (Toluol) = 462 nm, Quantenausbeute (Toluol): 35 %; λ_{max, em} (Film) = 472 nm

### 8-Naphthyl-2-yl-7,10-diphenyl-fluoranthen: (nicht erfindungsgemäß)

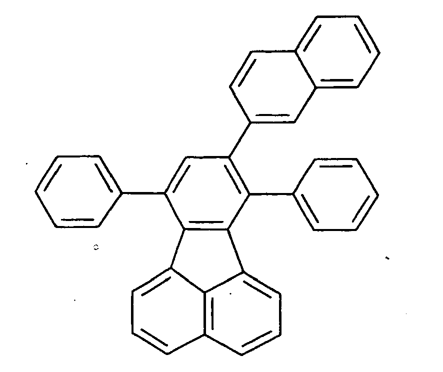

1,281 g 1-Ethinylnaphthalin und 3 g 7,9-Diphenyl-cyclpenta[a]acenaphthylen-8-on (Acecyclon, synthetisiert nach Dilthey et al., J. prakt. Chem. 1935, 143, 189) wurden in 20 g Xylol gelöst und 16 h unter Rückfluss erhitzt. Fällen in Methanol und Chromatographie an Kieselgel (Merck Kieselgel 60, Essigester/Cyclohexan) ergaben 3,1 g eines beigen Feststoffs.
λ_{max,em} (Toluol) = 468 nm, Quantenausbeute (Toluol): 31 %, λ_{max,em} (Film) = 466 nm

### 8-Nonyl-9-octyl-7,10-diphenyl-fluoranthen: (nicht erfindungsgemäß)

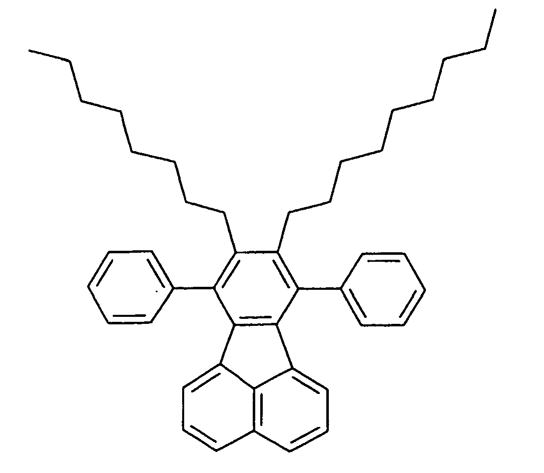

1,484 g 9-Nonadecin und 2 g Acecyclon wurden in 15 g Diphenylether gelöst und 16 h unter Rückfluß erhitzt. Fällen in Methanol und Chromatographie an Kieselgel (Merck Kieselgel 60, Essigester/Cyclohexan) ergaben 8-Nonyl-9-octyl-7,10-diphenyl-fluoranthen als bräunlichen Feststoff.
λmax_{, em} (Toluol) = 468 nm, Quantenausbeute (Toluol): 21 %

### Benzol-1,4-bis-(2,9-diphenyl-fluoranth-1-yl):

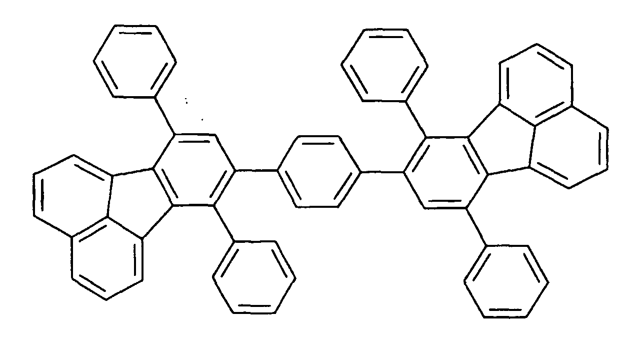

1 g 1,4-Diethinylbenzol und 6,5 g Acecyclon wurden in 22 g Xylol gelöst und 16 h unter Rückfluß erhitzt. Chromatographie an Kieselgel (Merck Kieselgel 60, Essigester/Cyclohexan) ergaben Benzol-1,4-bis(2,9-diphenyl-fluoranth-1-yl) als gelblichen Feststoff.
λmax_{, em} (Toluol) = 461 nm, Quantenausbeute (Toluol): 59 %; λ_{max, em} (Film) = 467 nm

### Benzol-1,3,5-tris-(2,9-diphenyl-fluoranth-1-yl):

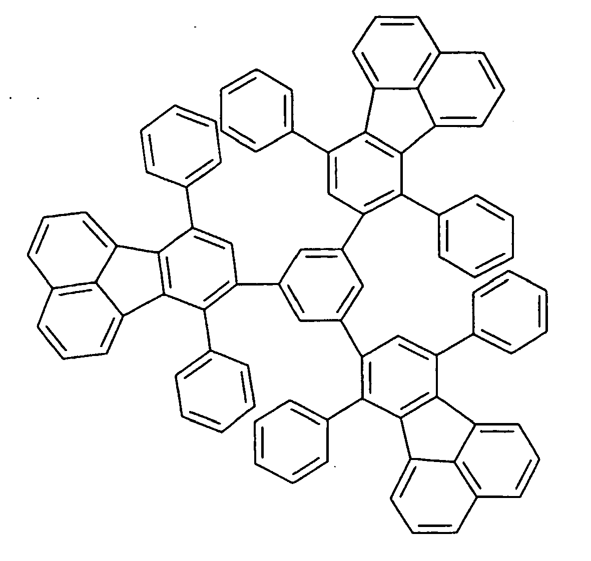

0,2 g 1,3,5-Triethinylbenzol und 2 g Acecyclon wurden in 20 g Xylol gelöst und 24 h unter Rückfluss erhitzt. Fällen in Methanol und Filtration über Kieselgel (Merck Kieselgel 60, Essigester/Cyclohexan) ergaben 0,6 g eines beigen Feststoffs.
λmax_{, em} (Toluol) = 459 nm, Quantenausbeute (Toluol): 51 %; λ_{max, em} (Film) = 467 nm

### 9,9'-Dimethyl-7,10,7',10'-tetraphenyl-[8,8']bifluoranthen:

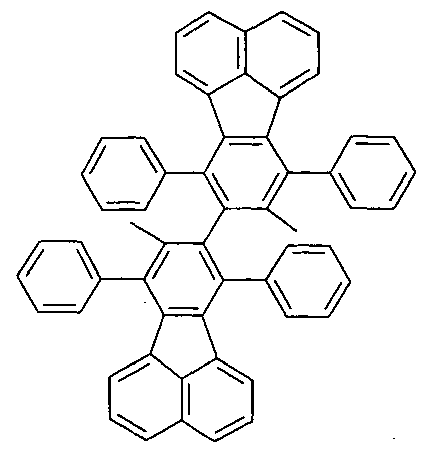

0,61 g 2,4-Hexadiin und 8 g Acecyclon wurden in 15 g Diphenylether gelöst und 26 h unter Rückfluss erhitzt. Abdestillieren des Lösungsmittels und Chromatographie an Kieselgel (Merck Kieselgel 60, Essigester/Cyclohexan) ergaben 4,2 g eines beigen Feststoffs.
λmax_{, em} (Toluol) = 463 nm, Quantenausbeute (Toluol): 34 %

### 9,10-Bis(2,9,10-triphenyl-fluoranthen-1-yl)anthracen, 9,10-Bis(9,10-diphenyl-2-octylfluoranthen-1-yl)anthracen:

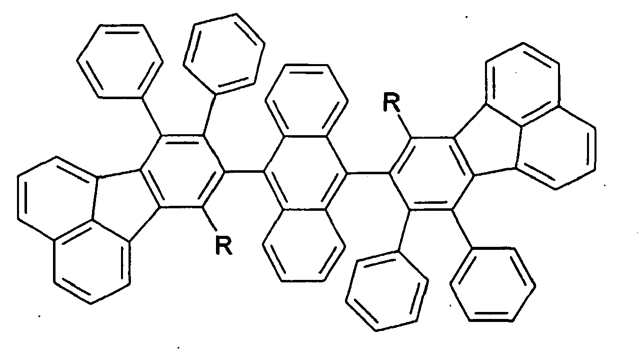

R = Phenyl, Octyl

Phenylderivat: 0,92 g 9,10-Bis-phenylethinyl-anthracen und 2 g Acecyclon wurden in 15 g Diphenylether gelöst und 14 h unter Rückfluss erhitzt. Abdestillieren des Lösungsmittels und Fällen in Methanol ergaben 0,7 g eines grauen Feststoffs.
λmax_{, em} (Toluol) = 456 nm, Quantenausbeute (Toluol): 72 %, λ_{max, em} (Film) = 461 nm

Alkylderivat: 0,91 g 9,10-Bis-(4-octyl-phenylethinyl)-anthracen (synthetisiert durch zweifache Pd(0)-Kupplung nach Hagihara-Sonogashira von 1-Decin an 9,10-Dibromoanthracen (gemäß Hagihara et al., Synthesis 1980, 627) und 2,1 g Acecyclon wurden in 15 g Diphenylether gelöst und 10 h unter Rückfluss erhitzt. Abdestillieren des Lösungsmittels, Fällen in Ethanol und Chromatographie an Kieselgel (Merck Kieselgel 60, Essigester/Cyclhexan) ergaben 9,10-Bis(9,1 10-diphenyl-2-octyl-fluoranthen-1-yl)anthracen (1,8 g).
λmax_{, em} (Toluol) = 455 nm, Quantenausbeute (Toluol): 44 %

## Patentansprüche

1. Fluoranthenderivate der allgemeinen Formel I 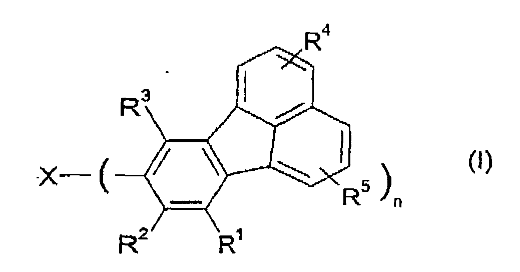 worin die Symbole die folgenden Bedeutungen aufweisen:
R¹, R², R³, R⁴, R⁵ Wasserstoff, ein Alkylrest, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
X ein Alkylrest ein aromatischer Rest, ein kondensiertes aromatisches Ringsystem, ein heteroaromatischer Rest oder ein Rest der Formel (I') 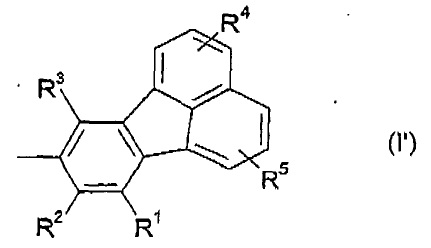 oder eine Oligophenylgruppe;
n 2 oder 3, oder im Falle von X = Oligophenylgruppe, 1 - 20;
unter der Bedingung, dass R¹, R², R³ und X nicht gleichzeitig einen Phenylrest bedeuten, wenn R⁴ und R⁵ Wasserstoff sind.

2. Fluoranthenderivate nach Anspruch 1, **dadurch gekennzeichnet, dass** R⁴ und R⁵ Wasserstoff sind.

3. Fluoranthenderivate nach Anspruch 1 oder 2, **dadurch gekennzeichnet, dass** R¹ und R³ jeweils ein Phenylrest sind.

4. Fluoranthenderivate nach einem der Ansprüche 1 bis 3, **dadurch gekennzeichnet, dass** X ein aromatischer Rest, ein kondensiertes aromatisches Ringsystem oder ein Rest der Formel I' oder eine Oligophenylgruppe ist

5. Verfahren zur Herstellung von Fluoranthenderivaten nach einem der Ansprüche 1 bis 4 durch Umsetzung einer Verbindung der Formel II 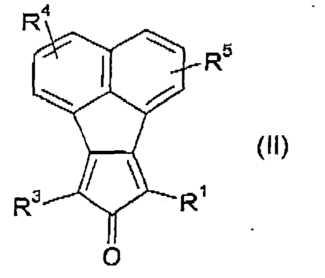 mit einer Alkinylverbindung der Formel III 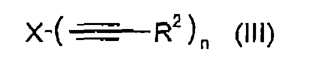 und anschließender Kohlenmonoxid-Eliminierung,
worin die Symbole die folgenden Bedeutungen aufweisen:
R¹, R², R³, R⁴, R⁵ Wasserstoff, ein Alkylrest, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist;
X ein Alkylrest, ein aromatischer Rest, ein kondensiertes aromatisches Ringsystem, ein heteroaromatischer Rest oder ein Rest der Formel (I') 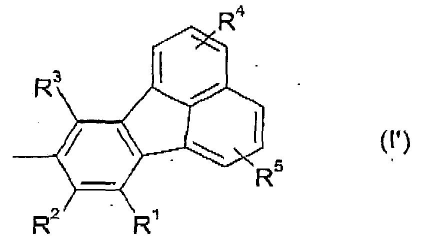 oder eine Oligophenylgruppe;
n 2 oder 3 oder, im Falle von X = Oligophenylgruppe, 1 bis 20.

6. Verfahren nach Anspruch 5, **dadurch gekennzeichnet, dass** die Verbindung der Formel (II) Acecyclon ist.

7. Verwendung von Fluoranthenderivaten der allgemeinen Formel (I) 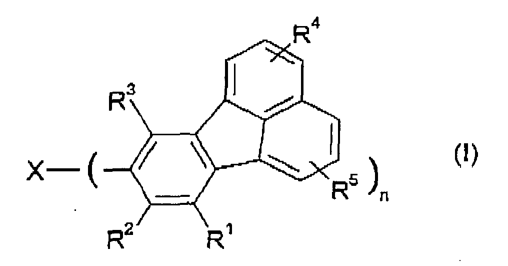 worin die Symbole die folgenden Bedeutungen ausweisen:
R¹, R², R³, R⁴, R⁵ Wasserstoff, ein Alkylrest, ein aromatischer Rest, ein kondensiertes aromatisches Ringsystem, ein heteroaromatischer Rest oder -CH=CH₂, (E)- oder (Z)-CH=CH-C₆H₅, Acryloyl, Methacryloyl, Methylstyryl, -O-CH=CH₂ oder Glycidyl;
wobei mindestens einer der Reste R¹, R² und/oder R³ nicht Wasserstoff ist,
X ein Alkylrest, ein aromatischer Rest, ein kondensiertes aromatisches Ringsystem, ein heteroaromatischer Rest oder ein Rest der Formel (I') 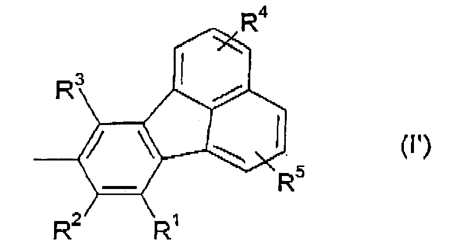 oder eine Oligophenylgruppe;
n 2 oder 3, im Fall von X = Oligophenylgruppe, 1 bis 20;
oder von Fluoranthenderivaten nach einem der Ansprüche 1 bis 5 als Emittermolekül in organischen lichtemittierenden Dioden (OLEDs).

8. Lichtemittierende Schicht enthaltend ein oder mehrere Fluoranthenderivate der allgemeinen Formel (I) nach einem der Ansprüche 1 bis 4 als Emittermoleküle.

9. OLED enthaltend eine lichtemittierende Schicht gemäß Anspruch 8.

10. Vorrichtung ausgewählt aus der Gruppe bestehend aus stationären Bildschinnen wie Bildschirmen von Computern, Fernsehern, Bildschirmen in Druckern, Küchengeräten sowie Reklametafeln, Beleuchtungen, Hinweistafeln und mobilen Bildschirmen wie Bildschirmen in Handys, Laptops, Fahrzeugen sowie Zielanzeigen an Bussen und Bahnen, enthaltend eine OLED gemäß Anspruch 9.

## Claims

1. A fluoranthene derivative of the general formula I 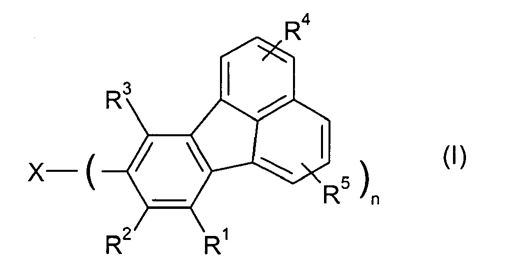 where the symbols have the following meanings:
R¹, R², R³, R⁴, R⁵ are each hydrogen, alkyl, an aromatic radical, a fused aromatic ring system, a heteroaromatic radical or -CH=CH₂, (E)- or (Z)-CH=CH-C₆H₅, acryloyl, methacryloyl, methylstyryl, -O-CH=CH₂ or glycidyl;
where at least one of the radicals R¹, R² and/or R³ is not hydrogen;
X is an alkyl radical, an aromatic radical, a fused aromatic ring system, a heteroaromatic radical or a radical of the formula (I') 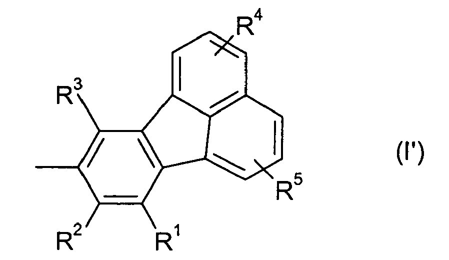 or an oligophenyl group;
n is 2 or 3 or, in the case of X = oligophenyl group, 1-20;
with the proviso that R¹, R², R³ and X are not at the same time phenyl when R⁴ and R⁵ are hydrogen.

2. The fluoranthene derivative according to claim 1, wherein R⁴ and R⁵ are each hydrogen.

3. The fluoranthene derivative according to claim 1 or 2, wherein R¹ and R³ are each a phenyl radical.

4. The fluoranthene derivative according to any of claims 1 to 3, wherein X is an aromatic radical, a fused aromatic ring system or a radical of the formula I' or an oligophenyl group.

5. A process for preparing fluoranthene derivatives according to any of claims 1 to 4 by reaction of a compound of the formula II 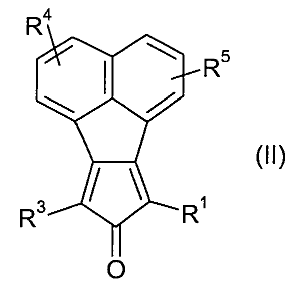 with an alkynyl compound of the formula (III) 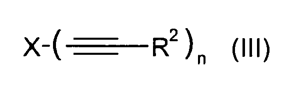 and subsequent elimination of carbon monoxide,
where the symbols have the following meanings:
R¹, R², R³, R⁴, R⁵ are each hydrogen, alkyl, an aromatic radical, a fused aromatic ring system, a heteroaromatic radical or -CH=CH₂, (E)- or (Z)-CH=CH-C₆H₅, acryloyl, methacryloyl, methylstyryl, -O-CH=CH₂ or glycidyl;
where at least one of the radicals R¹, R² and/or R³ is not hydrogen;
X is an alkyl radical, an aromatic radical, a fused aromatic ring system, a heteroaromatic radical or a radical of the formula (I') 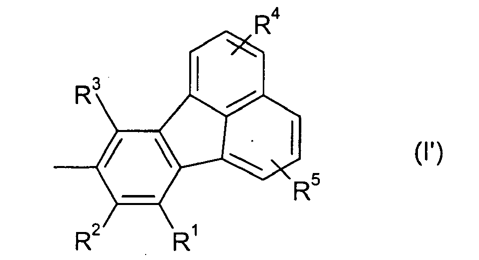 or an oligophenyl group;
n is 2 or 3 or, in the case of X = oligophenyl group, from 1 to 20.

6. The process according to claim 5, wherein the compound of the formula (II) is acecyclone.

7. The use of fluoranthene derivatives of the general formula (I) 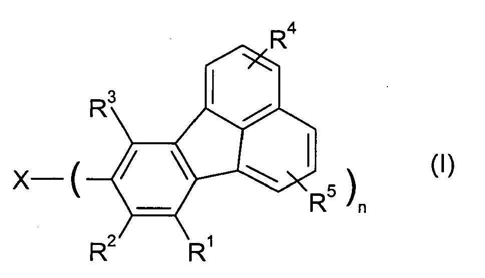 where the symbols have the following meanings:
R¹, R², R³, R⁴, R⁵ are each hydrogen, alkyl, an aromatic radical, a fused aromatic ring system, a heteroaromatic radical or -CH=CH₂, (E)- or (Z)-CH=CH-C₆H₅, acryloyl, methacryloyl, methylstyryl, -O-CH=CH₂ or glycidyl;
where at least one of the radicals R¹, R² and/or R³ is not hydrogen;
X is an alkyl radical, an aromatic radical, a fused aromatic ring system, a heteroaromatic radical or a radical of the formula (I') 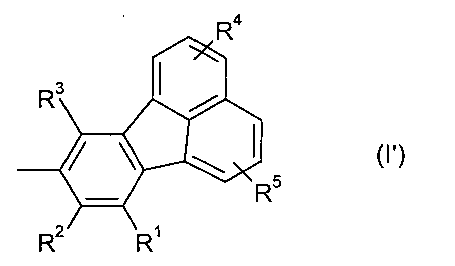 or an oligophenyl group;
n is 2 or 3 or, in the case of X = oligophenyl group, from 1 to 20;
or of fluoranthene derivatives according to any of claims 1 to 5 as emitter molecule in organic light-emitting diodes (OLEDs).

8. A light-emitting layer comprising one or more floranthene derivatives of the general formula (I) according to any of claims 1 to 4 as emitter molecules.

9. An OLED comprising a light-emitting layer according to claim 8.

10. A device selected from the group consisting of stationary VDUs such as VDUs of computers, televisions, VDUs in printers, kitchen appliances and advertising signs, lighting, information signs and mobile VDUs such as VDUs in mobile telephones, laptops, vehicles and destination displays on buses and trains comprising an OLED according to claim 9.

## Revendications

1. Dérivés de fluoranthène de formule générale I : 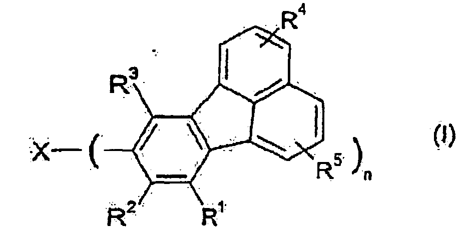 dans laquelle les symboles ont les significations suivantes :
R¹, R², R³, R⁴, R⁵ représentent l'hydrogène, un radical alkyle, un radical aromatique, un système cyclique aromatique condensé, un radical hétéroaromatique ou un groupement -CH=CH₂, (E)- ou (Z)-CH=CH-C₆H₅, acryloyle, méthacryloyle, méthylstyryle, -O-CH=CH₂ ou glycidyle ;
au moins l'un des radicaux R¹, R² et/ou R³ n'étant pas l'hydrogène ;
X représente un radical alkyle, un radical aromatique, un système cyclique aromatique condensé, un radical hétéroaromatique ou un radical de formule (I') : 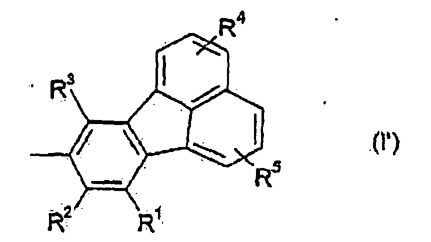 ou un groupement oligophényle ;
n vaut 2 ou 3 ou, dans le cas où X représente un groupement oligophényle, présente une valeur de 1 à 20 ;
à condition que R¹, R², R³ et X ne représentent pas simultanément un radical phényle, si R⁴ et R⁵ représentent l'hydrogène.

2. Dérivés de fluoranthène selon la revendication 1, **caractérisés en ce que** R⁴ et R⁵ représentent l'hydrogène.

3. Dérivés de fluoranthène selon la revendication 1 ou 2, **caractérisés en ce que** R¹ et R³ représentent, respectivement, un radical phényle.

4. Dérivés de fluoranthène selon l'une quelconque des revendications 1 à 3, **caractérisés en ce que** X représente un radical aromatique, un système cyclique aromatique condensé ou un radical de formule I' ou un groupement oligophényle.

5. Procédé pour fabriquer des dérivés de fluoranthène selon l'une quelconque des revendications 1 à 4, en faisant réagir un composé de formule II : 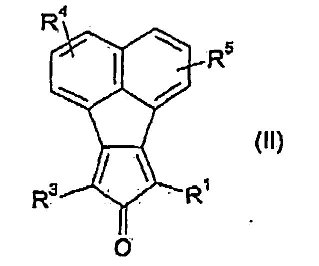 avec un composé d'alcynyle de formule III : 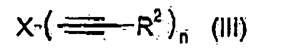 avec élimination suivante de monoxyde de carbone, dans lesquelles les symboles ont les significations suivantes :
R¹, R², R³, R⁴, R⁵ représentent l'hydrogène, un radical alkyle, un radical aromatique, un système cyclique aromatique condensé, un radical hétéroaromatique ou un groupement -CH=CH₂, (E) - ou (Z) -CH=CH-C₆H₅, acryloyle, méthacryloyle, méthylstyryle, -O-CH=CH₂ ou glycidyle,
au moins l'un des radicaux R¹, R² et/ou R³ ne représentant pas l'hydrogène ;
x représente un radical alkyle, un radical aromatique, un système cyclique aromatique condensé, un radical hétéroaromatique ou un radical de formule (I') : 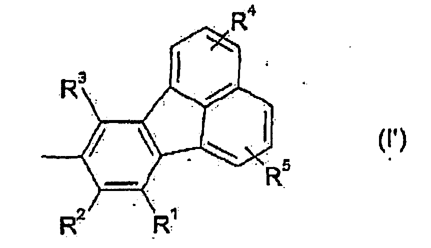 ou un groupement oligophényle ;
n vaut 2 ou 3 ou, dans le cas où X représente un groupement oligophényle, présente une valeur de 1 à 20.

6. Procédé selon la revendication 5, **caractérisé en ce que** le composé de formule (II) est l'acécyclone.

7. Utilisation de dérivés de fluoranthène de formule générale (I) : 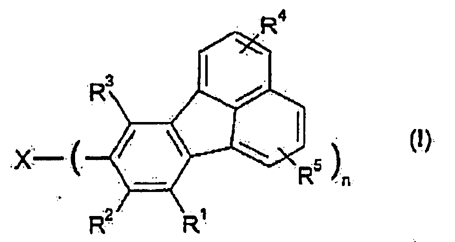 dans laquelle les symboles ont les significations suivantes :
R¹, R², R³, R⁴, R⁵ représentent l'hydrogène, un radical alkyle, un radical aromatique, un système cyclique aromatique condensé, un radical hétéroaromatique ou un groupement -CH=CH₂, (E)- ou (Z)-CH=CH-C₆H₅, acryloyle, méthacryloyle, méthylstyryle, -O-CH=CH₂ ou glycidyle ;
au moins l'un des radicaux R¹, R² et/ou R³ ne représentant pas l'hydrogène,
x représente un radical alkyle, un radical aromatique, un système cyclique aromatique condensé, un radical hétéroaromatique ou un radical de formule (I') : 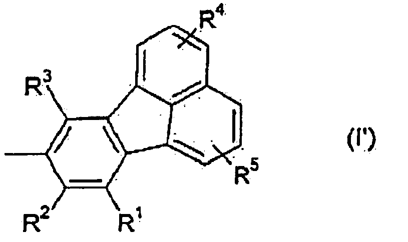 ou un groupement oligophényle ;
n vaut 2 ou 3 ou, dans le cas où X représente un groupement oligophényle, présente une valeur de 1 à 20 ;
ou de dérivés de fluoranthène selon l'une quelconque des revendications 1 à 5, comme molécule émettrice dans des diodes électroluminescentes organiques (DELO).

8. Couche électroluminescente contenant un ou plusieurs dérivés de fluoranthène de formule générale (I) selon l'une quelconque des revendications 1 à 4, comme molécule émettrice.

9. DELO contenant une couche électroluminescente selon la revendication 8.

10. Dispositif choisi dans le groupe constitué des écrans fixes, tels que les écrans d'ordinateurs, de téléviseurs, les écrans d'imprimantes, d'appareils ménagers, ainsi que les panneaux publicitaires, les éclairages, les panneaux indicateurs, et les écrans mobiles, tels que les écrans de téléphones portables, d'ordinateurs portables, de véhicules, ainsi que les affichages de destination sur les bus et les voies ferrées, contenant une DELO selon la revendication 9.
